# EUROPEAN PATENT SPECIFICATION

(11) **EP 3 601 670 B1**
(45) Date of publication and mention of the grant of the patent: **06.04.2022**
(21) Application number: 18723607.0
(22) Date of filing: 20.03.2018
(51) Int. Cl.: D21H 17/00, D21H 17/02, D21H 17/65, D06P 1/34, D21H 21/14, D21H 21/28, C12R 1/89

(54) **COMPOSITION COMPRISING CULTIVATED MICROALGAE FOR USE IN COLORING PROCESSES**
ZUSAMMENSETZUNG MIT KULTIVIERTEN MIKROALGEN ZUR VERWENDUNG IN FÄRBEVORGÄNGEN
COMPOSITION COMPRENANT DES MICROALGUES CULTIVÉES DESTINÉES À ÊTRE UTILISÉES DANS DES PROCÉDÉS DE COLORATION

(30) Priority: 20.03.2017 US 201762473549 P
(43) Date of publication of application: 05.02.2020
(73) Proprietor: Algalife Ltd., 4292000 Beit Yizhak (IL)
(72) Inventor: KREBS, Renana, 4292000 Beit Yizhak (IL)
(74) Representative: Pearl Cohen Zedek Latzer Baratz UK LLP
(86) International application number: PCT/IL2018/050320
(87) International publication number: WO 2018/173051

(56) References cited:
- CN-B- 103 045 478
- KR-B1- 101 413 874
- US-A- 5 643 585
- US-A1- 2012 270 303

## Description

### TECHNOLOGICAL FIELD

The present invention is directed to composition for use in the process of coloring surfaces, such as fabrics, comprising cultivated microalgae.

### BACKGROUND

Microalgae are microscopic unicellular organisms capable to convert solar energy to chemical energy via photosynthesis. They contain numerous bioactive compounds that can be harnessed for commercial use. The potential of microalgal photosynthesis for the production of valuable compounds or for energetic use is widely recognized due to their more efficient utilization of sunlight energy as compared with higher plants. Microalgae can be used to produce a wide range of metabolites such as proteins, lipids, carbohydrates, carotenoids or vitamins for health, food and feed additives, cosmetics and for energy production.

Nowadays, there are numerous commercial applications of microalgae such as microalgae can be used to enhance the nutritional value of food and animal feed owing to their chemical composition; they play a crucial role in aquaculture and they can be incorporated into cosmetics. Moreover, they are cultivated as a source of highly valuable molecules. For example, polyunsaturated fatty acid oils are added to infant formulas and nutritional supplements and pigments are important as natural dyes.

Microalgae have three fundamental attributes that can be converted into technical and commercial advantages. They are genetically a very diverse group of organisms with a wide range of physiological and biochemical characteristics; thus, they naturally produce many different and unusual fats, sugars, bioactive compounds, etc. They can cost-effectively incorporate the stable isotopes¹³C,¹⁵N and ²H into their biomass and thus into various compounds they produce. They comprise a large, unexplored group of organisms, and thus provide a virtually untapped source of products.

In recent years, microalgae apart from being used as single-cell proteins, they are projected as living-cell factories for the production of bio-fuels and various beneficial biochemicals used in food, aquaculture, poultry and pharmaceutical industries due to presence of different useful compounds (J. Algal Biomass Utln. 2012, 3 (4): 89-100).

The pigment content in microalgae is a specific feature of each species. Its evaluation is essential as an indirect measure of cell growth, as well as a parameter to check the trophic level of waters. Components of algae are frequently used in cosmetics as thickening agents, water-binding agents, and antioxidants. Some microalgal species are established in the skin care market, the main ones being *Arthrospira* and *Chlorella.* Microalgae extracts can be mainly found in face and skin care products (e.g., anti-aging cream, refreshing or regenerant care products, emollient and as an anti-irritant in peelers). Microalgae are also represented in sun protection and hair care products. Typical species that are used for cosmetics are *Chondrus crispus, Mastocarpus stellatus, Ascophyllum nodosum, Alaria esculenta, Spirulina platensis, Nannochloropsis oculata, Chlorella vulgaris* and *Dunaliella salina.* Microalgae are a polyphyletic and biochemically diverse assemblage of chlorophyll-a containing microorganisms capable of oxygenic photosynthesis that are predominantly found in aquatic environments with observed high levels of ultraviolet (UV) radiation.

Microalgal pigment has commercial uses as a natural food coloring and cosmetic ingredient. Some microalgae contain substantial amounts of Carotene (besides beta carotene). Other types of coloring appear in microalgae as well. Beta carotene is used as a food coloring (with a major application in providing the yellow color to margarine), as a food additive to enhance the color of the flesh of fish and the yolk of eggs, and to improve the health and fertility of grain-fed cattle. Natural Beta Carotene has physical properties that make it superior to synthetic. In particular, natural Beta Carotene is fat soluble.

Marine microalgae are organisms that live in complex habitats submitted to extreme conditions (for example, changes of salinity, temperature, nutrients, UV-Vis irradiation, etc.), therefore, they must adapt rapidly to the new environmental conditions to survive, producing a great variety of secondary (biologically active) metabolites, which cannot be found in other organisms.

Since the properties of microalgae is greatly influenced by their environment and culture conditions, for the purpose of preparing commercially standardized bulk reagents suitable for the industry there was a need for a method of growing microalgae in a cultivated manner which uses the outdoor sunlight as a source of energy. US5534417 discloses an apparatus for confined growth of microalgae in an assembly of vertical, transparent tubes through which nutrient and air is carried with carbon dioxide. The microalgae is periodically harvested from the tubes. US5643585 discloses a non-soluble particulate coloring material comprises dry ground red microalgae-derived material as the base coloring matter for use in the coloring of food products and cosmetics.

### GENERAL DESCRIPTION

The inventors of the present application have found that cultivated microalgae may be used for coloring surfaces, such as for example paper, a fiber, a fabric, a cloth, a garment, wool, silk, cotton, leather, polymeric material and any combination thereof.

Thus, the present invention provides a composition comprising at least one cultivated microalgae, at least one mordant, and at least one additive selected from at least one enzyme, at least one dye binder, and at least one thickening agent, or a combination thereof.

The invention further provides the use of a composition comprising at least one cultivated microalgae and at least one mordant, for coloring a surface selected from paper, fiber, yarn, fabric, cloth, garment, silk, wool, cotton, leather, polymeric material and any combination thereof.

In a further aspect the invention provides the use of a composition comprising at least one cultivated microalgae and at least one mordant for coloring a fiber or a fabric.

The invention also provides a process for the preparation of a composition, comprising the steps of: mixing at least one cultivated microalgae with at least one mordant, and at least one of at least one enzyme, at least one dye binder, at least one thickening agent and any combinations thereof.

In another one of its aspects, the invention provides a fiber comprising a composition of the invention. In another aspect, the invention provides a fabric comprising a composition of the invention.

In another aspect, the invention provides a process of coloring a fabric surface comprising the steps of providing a composition of the invention, and mixing said composition with a fabric. In some embodiments, said mixture of composition and fabric are heated to a temperature in the range of 30 - 100C. In another embodiments, said fabric is pretreated. In some embodiments, said pretreatment is cationization of said fabric (i.e. making said fabric impregnated with a cationic solution). In other embodiments, said composition of the invention is used to print a pattern on said fabric using a printer or a pattern mold. In some embodiments the printed fabric is baked in an over for fixing said pattern on said fabric.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

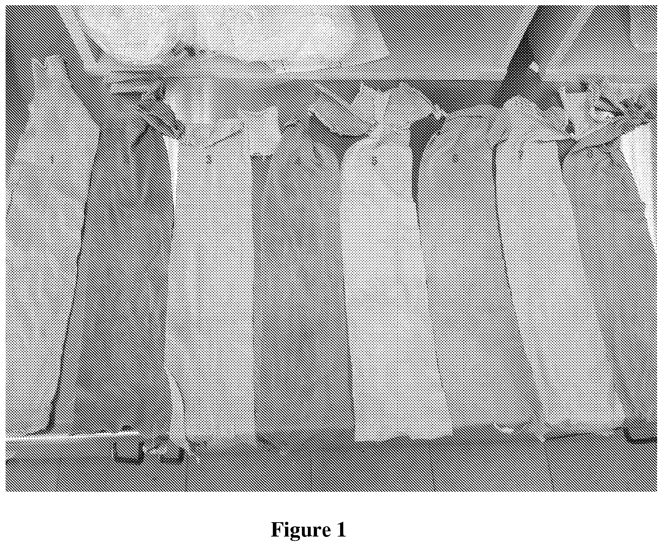

**Figure 1** shows the results of the coloring process using a composition of the invention as described in the example herein.

### DETAILED DESCRIPTION OF EMBODIMENTS

The invention provides a coloring and/or dyeing composition comprising at least one cultivated microalgae, at least one mordant, and at least one additive selected from at least one enzyme, at least one dye binder, and at least one thickening agent, or a combination thereof. The invention also provides the use of a coloring and/or dyeing composition comprising at least one cultivated microalgae and at least one mordant for coloring certain surfaces, fibers and fabrics.

The term ***"composition"*** as used in the context of the present invention should be understood to encompass any composition that is suitable for coloring and/or dyeing, i.e. transferring a color onto a surface, such as for example a fiber, a fabric and so forth.

The term ***"microalgae"*** should be understood to encompass are unicellular photosynthesis forming species which exist individually, or in chains or groups, having a size that can range from a few micrometers (µm) to a few hundreds of micrometers. Microalgae do not have roots, stems, or leaves. Microalgae biomass is typically measured with chlorophyll concentrations.

In some embodiments, said cultivated microalgae is selected from red algae, brown algae, green algae, red algae, blue algae and mixtures thereof. In other embodiments, said cultivated microalgae is a red microalgae. In yet further embodiments, said cultivated red microalgae is selected from the genus *Porphyridium, Rhodella, Hematococus (pluvialis), Dunaliella (bradawil)* and any mixture thereof.

The natural types of microalgae are usually found in freshwater and marine systems living in both the water column and sediment, however, the microalgae used in the present invention are ***"cultivated microalgae"*** thus, they are grown and cultured outside their natural marine environment, in regulated and controlled environments. In some embodiments, said cultivated microalgae are from a source that is other than a marine source. In further embodiments, said cultivated microalgae are non-naturally grown microalgae. In further embodiments, said cultivated microalgae are non-marine grown microalgae. In some other embodiments, said cultivated microalgae is grown/cultivated outside a marine environment (the cultivated microalgae used in the present invention are not harvested from a marine or natural water source). For example, in some embodiments, said cultivated microalgae are grown/cultivated in a microorganism growth apparatus. An example of a microorganism growth apparatus, including a process for growing said microalgae in the apparatus can be found in US5534417.

In some embodiments, a composition of the invention comprises at least 0.5% by weight of said at least one cultivated microalgae. In other embodiments, a composition of the invention comprises at least 1, 2, 3, 4, 5, 6, 7, 8, 9, 10, 11, 12, 13, 14, 15, 16, 17, 18, 19, 20% by weight of said at least one cultivated microalgae. In further embodiments, a composition of the invention comprises between about 5% to about 40% by weight of said at least one cultivated microalgae. In further embodiments, a composition of the invention comprises between about 1% to about 20% by weight of said at least one cultivated microalgae. In further embodiments, a composition of the invention comprises between about 5% to about 25% by weight of said at least one cultivated microalgae.

The term ***"mordant"*** as used herein should be understood to encompass any type of dye fixative, a substance used to set (i.e. bind) the coloring composition of the invention on a fiber and/or a fabric.

In some embodiments, said at least one mordant is from a natural source. In some embodiments, said at least one mordant is selected from Oak galls, Pomegranate rind Juniper needles and any combinations thereof. In other embodiments, said at least one mordant is from a synthetic source. selected from Tannic acid, Tartaric acid, Iron (II) sulfate heptahydrate, Aluminum potassium sulfate dodecahydrate, FeSO₄ and any combinations thereof. In other embodiments, said at least one mordant is tannic acid.

The composition of the invention further comprises at least one of the additive list consisting of: at least one enzyme (such as for example cellulose degrading enzyme), at least one dye binder (an agent that allows for the color composition to bind to the fiber of the fabric, such as AVCO BINDER D), at least one thickening agent (an agent that prevents the color composition from spreading by capillary attraction on the fabric or fibers) and any combinations thereof. In some embodiments, said at least one enzyme is a cellulose degradation enzyme.

Typical thickening agents are starch derivatives, flour, gum arabic, guar gumderivatives, tamarind, sodium alginate, sodium polyacrylate, gum Senegal and gum tragacanth, British gum or dextrine and albumen. Other thickeners include, alginatebased thickeners (such as AVCO-PRINT ESO) and cellulose based thickeners (such as THICKENER CM-7).

The present invention also provides the use of a composition comprising cultivated microalgae and at least one mordant, in a process of coloring a surface selected from paper, fiber, yarn, fabric, cloth, garment, silk, wool, cotton, leather, polymeric material and any combination thereof. When referring to a process of coloring a surface, it should be understood to encompass any type of coloring process including, dyeing and printing any type of color or pattern on a surface, thus endowing said surface with a color or a pattern with color.

In some embodiments, said process of coloring is dyeing (i.e. a process wherein the fabric, part of it or in its entirety, is immersed in the coloring composition of the invention and thus being endowed with a uniform color). In other embodiments, said process of coloring is printing (i.e. a process of applying color to fabric in definite patterns or designs. In printing, wooden blocks, stencils, engraved plates, rollers, or silkscreens or printers can be used to place the composition on the fabric).

In a further aspect the invention provides the use of a composition comprising cultivated microalgae and at least one mordant, in a process of coloring a fiber.

The term ***"fiber"*** should be understood to encompass a thread that can be used to make a yarn, fabric, knitted, woven, nonwoven fabric, cloth, paper, having a length longer than it is width.

In some embodiments, said fiber is a natural fiber. Such fibers include cellulose based fibers, protein-based fibers, mineral based fibers. Such natural fibers include but are not limited to: Seed fiber (fibers collected from seeds or seed cases. e.g. cotton and kapok, Leaf fiber (fibers collected from leaves. e.g., sansevieria, fique, sisal, banana and agave), Bast fiber (fibers are collected from the skin or bast surrounding the stem of their respective plant. These fibers have higher tensile strength than other fibers. Therefore, these fibers are used for durable yarn, fabric, packaging, and paper. Some examples are flax, jute, kenaf, industrial hemp, ramie, rattan, and vine fibers), skin fiber, fruit fiber (fibers are collected from the fruit of the plant, e.g. coconut (coir) fiber), stalk fiber (fibers that are actually the stalks of the plant. e.g. straws of wheat, rice, barley, and other crops including bamboo and grass, tree wood), animal fibers (generally comprise proteins such as collagen, keratin and fibroin; examples include silk, sinew, wool, catgut, angora, mohair and alpaca, animal hair (wool or hairs): fiber or wool taken from animals or hairy mammals. e.g. sheep's wool, goat hair (cashmere, mohair), alpaca hair, horse hair, etc. silk fiber: fiber secreted by glands (often located near the mouth) of insects during the preparation of cocoons, avian fiber: fibers from birds, e.g. feathers and feather fiber).

In other embodiments, said fiber is a manufactured fiber. Such fibers include, but are not limited to natural sugar based manufactured fibers - PLA, chitosan, protein based - rubber, azlon, cellulose based - rayon, lyocell, acetate, triacetate, synthetic polymers such as - acrylic, anidex, aramid, elastoester, fluoropolymer, lastrile, melamine, modacrylic, novoloid, nylon, nytril, olefin, PBI, polyester, rubber (synthetic), saran, spandex, sulfar, vinal, vinyon.

In other embodiments said fiber is a synthetic or artificial fiber (such as for example Nylon, Olefin, Acrylic, Polyester, Rayon, artificial silk, Vinyon, Saran, Spandex, Vinalon, Aramids, Nomex, Kevlar, Twaron, Modal, Dyneema/Spectra, PBI (Polybenzimidazole fiber), Sulfar, Lyocell, PLA, M-5 (PIPD fiber), Orlon, Zylon (PBO fiber), Vectran (TLCP fiber) made from Vectra LCP polymer, Derclon, Acrylonitrile rubber, Glass fiber, metallic fiber, expanded polystyrene flakes, urea-formaldehyde foam resin, polyurethane foam, phenolic resin foam and any combinations thereof.

The invention further provides the use of a composition comprising at least one cultivated microalgae and at least one mordant in a process for coloring a fabric. In some embodiments, said fabric is selected from a woven fabric, a nonwoven fabric, a knitted fabric or any combinations thereof.

In some other embodiments, the composition further comprises at least one further agent selected from an anti-irritating agent, an anti-microbial agent, vitamins, anti-septic, anti-inflammatory, anti-oxidants, an anti-sceptic agent, a softening agent.

In some embodiments, said fabric is used in the preparation of a garment. In other embodiments, said fabric is used in the preparation of a medical fabric (such as for example a bandage, a wound dressing, a casting form, a pressure wound dressing, an hygienic napkin, a sanitary napkin, burn dressing and so forth). In some embodiments, said fabric is used in the preparation of a wearable medical device.

The invention further provides a process for the preparation of a composition of the invention, comprising the steps of: mixing at least one cultivated microalgae with at least one at least one mordant (fixating agent), and at least one of at least one enzyme, at least one binding agent, a thickening agent and any combinations thereof. Optionally, at least one wetting agent may be included.

In a further aspect the invention provides a fiber comprising a composition of the invention. In a further aspect the invention provides a fabric comprising a composition of the invention.

### EXPERIMENTAL SECTION

***Cultivated Microalgae:*** Red microalgae of the type *Porphyridium sp.* The microalgae were cultivated under controlled conditions as disclosed in US5534417 and provided as biomass (either in a liquid form, a frozen biomass form or a powder).

### Preparing the dyeing composition for cellulose based fabrics

400 ml of cultivated microalgae biomass was mixed with 200 ml of distilled water. 12g of tannic acid (mordant) was added to the mixture.

### Preparing the cellulose based fabric for dyeing

Cationization solution: a mixture of 200 ml water, 1.2g AVCOPRET Ultra Cat.

Cationization process: The solution was added with a fabric sample (20g of cellulose based fabrics such as modal, bamboo or cotton) to a container which was heated in an oven for 20 minutes at 50C. After additional 50 minutes the fabrics were taken out of the container.

### Dyeing process for cellulose based fabrics

The process was performed in container of 1 liter. 600 ml of the dyeing composition was added to the container together with 20g of fabric that has been prepared by the cationization process.

The container was introduced into an oven for 30 minutes at 80C. After 30 minutes the oven was stopped. The container was opened, and small piece of the fabric were taken. The color of sample was pink. Then the container was entered into the over again for another 20 minutes at 98 C. The oven was stopped, and another sample of the fabric was taken, having beige/brown color. After taking the fabric out of the container it was dried.

### Preparing the dyeing composition for synthetic fabrics

***Fabrics:*** Nylon - Semi - Dull
***Materials:*** Enzyme - Avco - Zim und 3548, Mordant - Tannic acid, Biomass - Red micro algae, Biomass - Blue / Green micro algae. Microalgae were cultivated under controlled conditions as disclosed in US5534417.

Eight types of compositions were prepared:
Composition 1: Red Biomass (50 ml), Enzyme (50 ml), water (100 ml)
Composition 3: Red Biomass (50 ml), No Enzyme, water (100 ml)
Composition 5: Blue/Green Biomass (50 ml), Enzyme (50 ml), Water (100 ml)
Composition 7: Blue/Green Biomass (50 ml), No Enzyme, Water (100 ml)
Composition 2: Red Biomass (50 ml), Enzyme (50 ml), Mordant (50 ml), water (100 ml)
Composition 4: Red Biomass (50 ml), No Enzyme, Mordant (50 ml), Water (100 ml)
Composition 6: Blue/Green Biomass (50 ml), Enzyme (50 ml), Mordant (50 ml), Water (100 ml).
Composition 8: Blue/Green Biomass (50 ml), No Enzyme, Mordant (50 ml), Water (100 ml).

Each composition was added to a container with fabric sample and entered into an oven for 120 minutes in 98C. At the end of the oven treatment the fabric samples were taken out of the container and rinsed twice (10 minutes with warm water at 40C and another 10 minutes with tap cold water). The fabrics were then spinned and washed in a washing machine. Figure 1 shows the coloring results. Table 1 below describes the results.

**Table 1 - Results of coloring compositions 1 - 8 above**

| **Sample number** | **Pigment and other materials** | **Color recieved** |
|---|---|---|
| (1) | Red with enzyme | Beige |
| (3) | Red with no enzyme | Light Beige |
| (2) | Red with enzyme and with mordant | Green-Brown |
| (4) | Red with no enzyme with mordant | Green-Blue |
| (5) | Green/Blue with enzyme | Lemon green |
| (7) | Green/Blue with no enzyme | Lemon green |
| (6) | Green/Blue with enzyme and with mordant | Green-Brown |
| (8) | Green/Blue with no enzyme with mordant | Dark Green-Brown |

As can be understood from the table above, the compositions wherein mordant was added provided a fabric that was colored with stable color that did not wash off.

### Preparing the coloring composition for printing on fabrics

Materials - Biomass of red micro algae (frozen) (cultivated under controlled conditions as disclosed in US5534417), Enzyme: Avco - Zim und 3548, Water: distillated water, Binder: Avcobinder D, Fixator: UP. Fabrics - Modal after washing in washing machine, Cotton - P.G. 76/68 677327

30g of frozen biomass was melted, and mixed with 125 ml water, 15 ml enzyme. The mixture was stirred for 5 hours at 40C. To the mixture 48 ml binder and 3 ml fixator were added.

The composition was applied to the fabric by hand printing and drying the printed fabrics with warm air. Each printed fabric was baked in an oven heated to 120C for 2 minutes.

## Claims

1. A composition comprising at least one cultivated microalgae; at least one mordant; and at least one additive selected from at least one enzyme, at least one dye binder, and at least one thickening agent, or a combination thereof.

2. A composition according to claim 1, wherein said at least one cultivated microalgae is selected from red algae, brown algae, green algae, red algae, blue algae and any combinations thereof.

3. A composition according to claims 1 or 2, wherein said at least one cultivated microalgae is a red microalgae.

4. A composition according any one of the preceding claims, wherein said cultivated microalgae comprises at least 0.5% of said composition.

5. A composition according to any one of the preceding claims, wherein said at least one mordant is selected from Tannic acid, Tartaric acid, Iron (II) sulfate heptahydrate, Aluminum potassium sulfate dodecahydrate, FeSO₄ and any combinations thereof.

6. A composition according to any one of the preceding claims, wherein said at least one mordant is tannic acid.

7. Use of a composition comprising at least one cultivated microalgae and at least one mordant for coloring a surface selected from paper, fiber, yarn, fabric, cloth, garment, silk, wool, cotton, leather, polymeric material and any combination thereof.

8. Use of a composition comprising at least one cultivated microalgae and at least one mordant in coloring a fiber or a fabric.

9. Use according to claim 7 or 8 wherein the composition is as defined in any of claims 1 to 6.

10. A process for the preparation of a composition according to any one of claims 1 to 6, comprising the steps of: mixing at least one cultivated microalgae with at least one mordant and at least one of at least one enzyme, at least one wetting agent, at least one dye binder, at least one thickening agent and any combinations thereof.

11. A fiber comprising a composition according to any one of claims 1 to 6.

12. A fabric comprising a composition according to any one of claims 1 to 6.

13. A process of coloring a fabric comprising the steps of providing a composition according to any of claims 1 to 6, and mixing said composition with a fabric.

14. A process of claim 13, wherein said mixture of composition and fabric are heated to a temperature in the range of 30 - 100°C.

## Patentansprüche

1. Zusammensetzung, umfassend mindestens eine kultivierte Mikroalge; mindestens ein Beizmittel; und mindestens ein Additiv, das aus mindestens einem Enzym, mindestens einem Farbstoffbinder und mindestens einem Verdickungsmittel oder einer Kombination davon ausgewählt ist.

2. Zusammensetzung nach Anspruch 1, wobei die mindestens eine kultivierte Mikroalge aus Rotalgen, Braunalgen, Grünalgen, Rotalgen, Blaualgen und beliebigen Kombinationen davon ausgewählt ist.

3. Zusammensetzung nach Anspruch 1 oder 2, wobei die mindestens eine kultivierte Mikroalge eine rote Mikroalge ist.

4. Zusammensetzung nach einem der vorhergehenden Ansprüche, wobei die kultivierte Mikroalge mindestens 0,5 % der Zusammensetzung umfasst.

5. Zusammensetzung nach einem der vorhergehenden Ansprüche, wobei das mindestens eine Beizmittel aus Gerbsäure, Weinsteinsäure, Eisen(II)sulfat-Heptahydrat, Aluminiumkaliumsulfat-Dodecahydrat, FeSO₄ und beliebigen Kombinationen davon ausgewählt ist.

6. Zusammensetzung nach einem der vorhergehenden Ansprüche, wobei das mindestens eine Beizmittel Gerbsäure ist.

7. Verwendung einer Zusammensetzung, umfassend mindestens eine kultivierte Mikroalge und mindestens ein Beizmittel zum Kolorieren einer Oberfläche, die aus Papier, Textilfaser, Garn, Gewebe, Stoff, Bekleidung, Seide, Wolle, Baumwolle, Leder, Polymermaterial und einer beliebigen Kombination davon ausgewählt ist.

8. Verwendung der Zusammensetzung, umfassend mindestens eine kultivierte Mikroalge und mindestens ein Beizmittel beim Kolorieren einer Textilfaser oder eines Gewebes.

9. Verwendung nach Anspruch 7 oder 8, wobei die Zusammensetzung derart ist, wie sie in einem der Ansprüche 1 bis 6 definiert ist.

10. Prozess zur Herstellung einer Zusammensetzung nach einem der Ansprüche 1 bis 6, umfassend die folgenden Schritte: Mischen mindestens einer kultivierten Mikroalge mit mindestens einem Beizmittel und mindestens einem von einem Enzym, mindestens einem Benetzungsmittel, mindestens einem Farbstoffbinder, mindestens einem Verdickungsmittel und beliebigen Kombinationen davon.

11. Textilfaser, umfassend eine Zusammensetzung nach einem der Ansprüche 1 bis 6.

12. Gewebe, umfassend eine Zusammensetzung nach einem der Ansprüche 1 bis 6.

13. Prozess zum Kolorieren eines Gewebes, umfassend die Schritte zum Bereitstellen einer Zusammensetzung nach einem der Ansprüche 1 bis 6 und Mischen der Zusammensetzung mit einem Gewebe.

14. Prozess nach Anspruch 13, wobei das Gemisch aus Komposition und Gewebe auf eine Temperatur im Bereich von 30-100 °C erhitzt wird.

## Revendications

1. Composition comprenant au moins une microalgue cultivée ; au moins un mordant ; et au moins un additif choisi parmi au moins une enzyme, au moins un liant de colorant et au moins un agent épaississant, ou une combinaison de ceux-ci.

2. Composition selon la revendication 1, dans laquelle ladite au moins une microalgue cultivée est choisie parmi les algues rouges, les algues brunes, les algues vertes, les algues rouges, les algues bleues et toutes leurs combinaisons.

3. Composition selon les revendications 1 ou 2, dans laquelle ladite au moins une microalgue cultivée est une microalgue rouge.

4. Composition selon l'une quelconque des revendications précédentes, dans laquelle ladite microalgue cultivée comprend au moins 0,5 % de ladite composition.

5. Composition selon l'une quelconque des revendications précédentes, dans laquelle ledit au moins un mordant est choisi parmi l'acide tannique, l'acide tartrique, le sulfate de fer (II) heptahydraté, le sulfate d'aluminium et de potassium dodécahydraté, le FeSO₄ et toute combinaison de ceux-ci.

6. Composition selon l'une quelconque des revendications précédentes, dans laquelle ledit au moins un mordant est l'acide tannique.

7. Utilisation d'une composition comprenant au moins une microalgue cultivée et au moins un mordant pour colorer une surface choisie parmi du papier, une fibre, un fil, du tissu, une étoffe, un vêtement, de la soie, de la laine, du coton, du cuir, un matériau polymère et toute combinaison de ceux-ci.

8. Utilisation d'une composition comprenant au moins une microalgue cultivée et au moins un mordant pour colorer une fibre ou un tissu.

9. Utilisation selon la revendication 7 ou 8, dans laquelle la composition est telle que définie dans l'une quelconque des revendications 1 à 6.

10. Procédé de préparation d'une composition selon l'une quelconque des revendications 1 à 6, comprenant les étapes de : mélange d'au moins une microalgue cultivée avec au moins un mordant et au moins l'un parmi au moins une enzyme, au moins un agent mouillant, au moins un liant de teinture, au moins un agent épaississant et toute combinaison de ceux-ci.

11. Fibre comprenant une composition selon l'une quelconque des revendications 1 à 6.

12. Tissu comprenant une composition selon l'une quelconque des revendications 1 à 6.

13. Procédé de coloration d'un tissu comprenant les étapes de fourniture d'une composition selon l'une quelconque des revendications 1 à 6, et de mélange de ladite composition avec un tissu.

14. Procédé selon la revendication 13, dans lequel ledit mélange de composition et de tissu est chauffé à une température dans une plage de 30 à 100°C.
